# FASCICULE DE BREVET EUROPEEN

(11) **EP 1 163 049 B1**
(45) Date de publication et mention de la délivrance du brevet: **25.05.2005**
(21) Numéro de dépôt: 00909431.9
(22) Date de dépôt: 06.03.2000
(51) Int. Cl.: B01J 19/00, C07H 21/00

(54) **PROCEDE DE REALISATION D'UNE MATRICE DE SEQUENCES DE MOLECULES CHIMIQUES OU BIOLOGIQUES POUR DISPOSITIF D'ANALYSE CHIMIQUE OU BIOLOGIQUE**
VERFAHREN ZUR HERSTELLUNG EINER MATRIZE VON CHEMISCHEN ODER BIOLOGISCHEN MOLEKÜLSEQUENZEN FÜR EINE CHEMISCHE ODER BIOLOGISCHE ANALYSEVORRICHTUNG
METHOD FOR PRODUCING A MATRIX OF SEQUENCES OF CHEMICAL OR BIOLOGICAL MOLECULES FOR A CHEMICAL OR BIOLOGICAL ANALYSIS DEVICE

(30) Priorité: 08.03.1999 FR 9902819
(43) Date de publication de la demande: 19.12.2001
(73) Titulaire: COMMISSARIAT A L'ENERGIE ATOMIQUE, 75752 Paris Cédex 16 (FR)
(72) Inventeur: ROSILIO, Charles, F-91190 Gif-sur-Yvette (FR); VINET, Françoise, F-38000 Grenoble (FR); VAUCHIER, Claude, F-38120 Saint-Egreve (FR); CLERC, Jean-Frédéric, F-38120 Le Fontanil (FR)
(74) Mandataire: Audier, Philippe André
(86) Numéro de dépôt international: PCT/FR2000/000550
(87) Numéro de publication internationale: WO 2000/053310

(56) Documents cités:
- EP-A- 0 728 520
- WO-A-95/35505
- DE-A- 19 543 232
- US-A- 5 658 734
- US-A- 5 763 263

## Description

### Domaine technique

La présente invention a pour objet un procédé de réalisation en parallèle d'une matrice de séquences de molécules chimiques ou biologiques pour dispositif d'analyse chimique ou biologique.

Les molécules chimiques ou biologiques peuvent être en particulier des aminoacides ou des nucléotides, les séquences étant alors des peptides ou des oligonucléotides.

Elle s'applique en particulier à la réalisation d'une matrice de sondes d'oligonucléotides de différentes séquences pour l'analyse ou le diagnostic ou la recherche en génétique (mutations, polymorphismes, transcriptome).

### État de la technique antérieure

De nouveaux systèmes ou dispositifs d'analyse chimique ou biologique sont utilisés pour le séquençage et l'étude de l'expression des gènes. Ils sont constitués d'un ensemble de sondes moléculaires (oligonucléotides) fixées sur la surface miniaturisée d'un substrat afin de réaliser une biopuce ou puce à ADN.

Au cours de l'analyse d'un échantillon, les acides nucléiques cibles extraits sont marqués et déposés sur la matrice de sondes. L'hybridation (appariement entre les brins complémentaires de la double hélice d'ADN) entre les sondes et les cibles marquées permet de repérer et d'identifier les séquences de l'échantillon d'ADN.

De nombreux procédés de réalisation ont été décrits et développés pour améliorer la miniaturisation et la capacité de densité de sites d'analyse sur la puce.

Différentes techniques de greffage et d'immobilisation des sondes (par liaison covalente ou électrostatique) sur différents substrats (silicium, verre, polymère, électrodes en or ...) ont fait l'objet de recherches et de développements industriels.
Les matrices de sondes sont alors réalisées
- soit par l'immobilisation d'oligonucléotides présynthétisés par greffage de ceux-ci sur un substrat fonctionnalisé ou sur un polymère conducteur,
- soit par synthèse in situ des oligonucléotides sondes sur le substrat.

Les procédés de synthèse in situ utilisés jusqu'à présent font appel à deux modes d'adressage différents des sites, qui sont soit l'adressage manuel par microrobot comme il est décrit dans US-A-5 474 796 [1], et l'adressage photochimique comme il est décrit dans WO-A-97/39 151 [2].

Dans le document US-A-5 474 796 [1], on forme tout d'abord sur le substrat des sites fonctionnalisés en utilisant des photorésists ou des masques pour définir les différents sites du substrat, puis on forme les séquences d'oligonucléotides sur les sites fonctionnalisés par injection des réactifs sur les sites voulus au moyen d'une pompe piézo-électrique.

Dans le document WO-A-97/39151 [2], on soumet tout d'abord le substrat à un traitement pour le munir de groupes fonctionnels protégés avec un groupe protecteur photolabile, puis on déprotège certains groupes fonctionnels du substrat par irradiation sélective à travers un masque pour définir les sites et on fait réagir ensuite sur ces sites un nucléotide pour construire la séquence voulue.

Dans les deux cas, la synthèse in situ met en jeu les réactions classiques de couplage par l'intermédiaire des phosphoramidites, des phosphites ou des phosphonates, pour la condensation successive des nucléotides judicieusement protégés. Le cycle de synthèse comprend les étapes de déprotection, couplage, blocage et oxydation, et permet de faire croître l'oligonucléotide à partir de la surface de chaque site.

Dans l'adressage photochimique, on réalise les étapes de déprotection des nucléotides par la lumière. L'adressage des sites se fait par une étape de lithographie (insolation au travers d'un masque). Le groupement protecteur photolabile est éliminé par la lumière, permettant ainsi de réaliser le couplage ultérieur par trempage du substrat dans une solution d'un nucléotide activé (par exemple d'un phosphoramidite), en présence d'un catalyseur, par exemple le tétrazole.

Ce procédé de synthèse combinatoire dirigé par la lumière nécessite la réalisation d'un nombre de masques égal au nombre de nucléotides qui composent l'oligonucléotide et constitue une opération coûteuse et fastidieuse. Si ce procédé présente l'avantage de conduire à des puces de très haute densité (résolution du procédé de photolithographie), le rendement des réactions photochimiques (élimination des groupements photolabiles) n'est par contre jamais de 100 %, et on ne peut être sûr de la pureté de la séquence oligonucléotidique réalisée sur la puce.

Dans le cas du document US-A-5 474 796 [1], qui utilise la technique d'impression par jets (têtes piézo-électriques) pour distribuer sur les différents sites de la puce, les 4 nucléotides activés de base de l'ADN ainsi que les réactifs de couplage, la synthèse en phase solide sur des sites d'une centaine de µm², avec des quantités de réactifs de l'ordre du nanolitre, exige des conditions d'atmosphère contrôlée (sensibilité à l'humidité et l'oxygène) difficiles à réaliser dans l'environnement d'un microdispenseur et de ses équipements associés. Elle nécessite par ailleurs des solvants à haut point d'ébullition et des réactifs peu volatils.

On connaît encore des procédés de synthèse in situ d'oligonucléotides utilisant des masques pour définir les zones du substrat qui doivent être soumises aux cycles successifs de synthèse.

Ainsi le document EP-A-0 728 520 [3] décrit un procédé de réalisation de matrices de sondes d'oligonucléotides utilisant un substrat comportant sur toute sa surface des groupes fonctionnels protégés, destinés à l'accrochage des nucléotides, et un matériau barrière pour définir les zones du substrat à modifier. Ce matériau peut être choisi parmi les laques, les huiles liquides, les polyesters, les polyuréthannes et les résines époxy qui sont généralement des prépolymères liquides. Il est déposé par différentes techniques d'impression. Après dépôt de la barrière, on procède à la déprotection en phase vapeur des groupes fonctionnels non recouverts du matériau barrière. L'utilisation d'une phase vapeur est nécessaire car le matériau barrière utilisé est soluble ou entraîné dans les solvants de déprotection. Mais la protection en phase vapeur pose des problèmes de risque de toxicité dans l'environnement et d'attaque par diffusion de matériaux.

Le document US-A-5 658 734 [4] utilise un substrat totalement fonctionnalisé et les techniques de lithographie avec emploi d'un photorésist pour définir les zones du substrat à modifier en intercalant entre le substrat et le photorésist, un polymère qui joue le rôle de tampon. Ce procédé utilise ainsi un photorésist qui doit être déposé à la tournette, sur la couche de polymère déposée sur le substrat, à chaque cycle additionnel d'un nucléotide, ce qui nécessite une étape de lithographie à chaque cycle (deux dépôts à la tournette, recuit thermique, insolation à travers un masque, développement de la résine photosensible, puis développement du polymère tampon). Après les étapes de déprotection des groupes fonctionnels du substrat et d'addition d'un nucléotide, à travers les ouvertures pratiquées dans le polymère, les deux couches sont éliminées dans un solvant. Dans ce procédé, le polymère permet d'isoler le photorésist de la couche biologique de nucléotide. Ce système nécessite donc en plus des étapes de dépôt à la tournette et de lithographie, la réalisation d'un grand nombre de masques.

Un autre inconvénient de ce procédé réside dans le risque de « lift off » de la couche protectrice au cours du développement du polymère protecteur. En effet par diffusion du solvant sous la couche de photorésist, on peut avoir un décollement du polymère sur les sites protégés.

La présente invention a précisément pour objet un procédé de réalisation d'une matrice de séquences de molécules chimiques ou biologiques, par exemple d'oligonucléotide, qui permet de pallier les inconvénients des procédés décrits ci-dessus.

### Exposé de l'invention

Le procédé de l'invention utilise un dépôt localisé sélectif d'un polymère protecteur sur un substrat structuré avec des microcuvettes fonctionnalisées. Le polymère protecteur est capable de protéger momentanément les microcuvettes choisies lors d'une réaction chimique conduisant au couplage avec la molécule de l'enchaînement suivant. Un adressage mécanique par microdéposition sur les microcuvettes choisies, remplace l'adressage photochimique ou électrique et les techniques de lithographie ou d'impression utilisées dans les autres procédés de synthèse combinatoire, par exemple pour la réalisation de sondes d'oligonucléotides.

Selon l'invention le procédé de réalisation d'une matrice de séquences de molécules chimiques ou biologiques formées d'enchaînements différents de molécules chimique ou M1, M2, ... Mn, par synthèse in situ sur un substrat structuré avec des microcuvettes, comprend les étapes suivantes :
1) fonctionnalisation des microcuvettes du substrat par des groupes fonctionnels capables de former une liaison covalente avec les molécules M1, M2, ... Mn ;
2) dépôt d'un polymère de protection dans au moins l'une des microcuvettes par microdéposition de gouttes dudit polymère pour constituer dans la(les) microcuvette(s) sélectionnée(s) des couvercles de polymère solide ;
3) réalisation d'une ou plusieurs réactions chimiques en phase liquide pour assurer le couplage d'une première molécule M1 sur les groupes fonctionnels des microcuvettes non recouvertes de polymère de protection ;
4) élimination du polymère de protection sur les microcuvettes recouvertes de ce polymère, après la première réaction ou l'une des réactions suivantes effectuées dans l'étape 3) ; et
5) réalisation à nouveau des étapes 2), 3) et 4) pour obtenir les séquences d'enchaînement voulu sur chacune des microcuvettes fonctionnalisées ;
dans lequel l'étape 1) de fonctionnalisation des microcuvettes consiste :
a) à fonctionnaliser la surface totale du substrat et des microcuvettes par les groupes fonctionnels, puis à protéger ces groupes fonctionnels par un groupe protecteur labile ;
b) à déposer un polymère de protection sur toutes les microcuvettes par microdéposition de gouttes dudit polymère pour constituer des couvercles de polymère solide sur toutes les microcuvettes ;
c) à déprotéger les groupes fonctionnels présents sur le substrat autour des microcuvettes, puis à les faire réagir avec un groupe bloquant non labile dans les conditions utilisées pour les réactions chimiques de couplage et pour l'élimination du polymère de protection ; et
d) à éliminer le polymère de protection de toutes les microcuvettes ;
et dans lequel le polymère de protection étant déposé à partir d'une solution, on recuit le film de polymère formé par chauffage du substrat à une température de 50 à 100°C avant d'effectuer l'étape 3) ou l'étape c).

Le procédé de l'invention permet ainsi par un choix judicieux du polymère de protection et d'un solvant de dissolution du polymère compatible avec les protocoles d'un automate de synthèse, de réaliser la synthèse de matrices d'oligonucléotides, dans des microcuvettes ou microréacteurs gravés dans un substrat. Pour ce faire, on associe un synthétiseur automatique d'oligonucléotides à un dispositif d'adressage mécanique (robot de microdéposition ou impression par jets d'encre) d'un polymère de protection.

L'adressage mécanique consiste à réaliser sélectivement un dépôt de polymère sur les microcuvettes choisies, constituant un couvercle solide et étanche ajusté sur les microcuvettes afin de délimiter et sélectionner des zones actives vis-à-vis des réactifs mis en oeuvre pour l'addition des unités monomères de l'enchaînement suivant.

Le procédé de l'invention donne la possibilité d'un choix de polymères protecteurs dont les propriétés de protection, d'insolubilité, d'étanchéité et de dépôt (forme et épaisseur contrôlées) sont compatibles et adaptées à l'une ou plusieurs étapes du cycle de synthèse.

Le procédé de l'invention permet d'éliminer le polymère de protection, soit après la première réaction chimique du cycle de couplage, soit après un cycle de couplage complet.

Le procédé de l'invention permet d'inclure l'élimination de ce couvercle en polymère dans le cycle classique de synthèse automatique des oligonucléotides, soit dans l'une des étapes de rinçage, soit dans une étape additionnelle de rinçage dans un solvant compatible avec ceux utilisés habituellement dans le synthétiseur automatique d'oligonucléotides (acétonitrile, dichlorométhane, tétrahydrofuranne).

De préférence, on réalise une protection des groupes fonctionnels des mïcrocuvettes lors de la mise en oeuvre du procédé de l'invention. Dans ce cas, on protège les groupes fonctionnels des microcuvettes par un groupe protecteur labile, et l'étape 3) comprend une première réaction de déprotection des groupes fonctionnels.

Selon l'invention, les molécules M1, M2, ... Mn peuvent être de différents types. A titre d'exemple, il peut s'agir d'acides aminés naturels ou synthétiques L- ou D-, de nucléotides (ARN ou ADN), de pentose ou d'hexose. Dans le cas des acides aminés le nombre de molécules M1, M2,... Mn peut être important. Dans le cas des nucléotides, le nombre de molécules est plus limité puisqu'il inclut A, T (ou U), G et C.

Dans ce cas, les nucléotides peuvent être sous forme de phosphoramidites, de phosphotriesters ou de H-phosphonates selon le type de synthèse nucléotidique mise en oeuvre. Ces molécules comportent deux fonctions réactives (hydroxyle et fonction avec phosphore) dont l'une, la fonction hydroxyle, est protégée par un groupe protecteur, de préférence identique au groupe protecteur des sites fonctionnalisés.

Un groupe protecteur approprié peut être en particulier un groupe trityle ou dérivé de trityle, par exemple le groupe diméthoxytrityle.

Lorsqu'on utilise le procédé de l'invention pour la réalisation de séquences d'oligonucléotides par synthèse phosphoramidite, l'étape 3) correspond à un cycle de synthèse et elle comprend l'élimination des groupes protecteurs trityle, le couplage avec un nucléotide, la réaction des groupes fonctionnels qui n'ont pas été couplés au nucléotide, avec un groupe bloquant, et l'oxydation en phosphate du groupement phosphoramidite du nucléotide.

Dans le procédé de l'invention, les étapes de déprotection (ou détritylation), de couplage, de blocage, d'oxydation et d'élimination du polymère de protection sont réalisées dans un synthétiseur automatique d'oligonucléotides en ayant introduit une modification de la chambre de réaction, permettant de traiter des substrats de taille variable.

Le groupe bloquant utilisé dans l'étape de blocage est un groupe non labile dans les conditions d'élimination des groupes protecteurs des groupes fonctionnels des microcuvettes.

A titre d'exemple, on peut utiliser pour cette réaction de blocage une solution de diméthylaminopyridine DMAP dans du tétrahydrofuranne et de la lutidine.

Dans le procédé de l'invention, les étapes de déprotection, de couplage, d'élimination du matériau de protection et éventuellement d'oxydation, sont réalisées par trempage du substrat, soit dans la chambre de réaction du synthétiseur, soit dans les bains de réactifs appropriés. Seule l'étape d'adressage des microcuvettes, qui correspond à une protection sélective des microcuvettes choisies par le polymère de protection est effectuée à l'aide de techniques de microdéposition.

Avantageusement, le polymère de protection est déposé sur les microcuvettes voulues à l'état liquide par adressage mécanique en utilisant des techniques de microdéposition sélective du type actuateur piézoélectriques, « pin and ring », impression par jets d'encres, vis d'Archimède et micropipettage.

L'adressage du polymère par microdéposition permet de protéger sélectivement des microcuvettes durant l'une ou plusieurs des quatre réactions de synthèse correspondant au couplage d'une unité nucléotidique. Il permet de distribuer localement sur les microcuvettes sélectionnées des gouttelettes calibrées d'un polymère en solution. Après évaporation du solvant, elles constituent des couvercles étanches ajustés sur les microcuvettes. Cet adressage se fait de préférence avant l'étape de détritylation ou avant l'étape de couplage dans le cycle de synthèse des oligonucléotides.

L'originalité du procédé réside dans les grandes possibilités d'adapter le choix du polymère et de son rôle protecteur aux différentes étapes de la synthèse des oligonucléotides.

Dans le procédé de l'invention, le choix du polymère protecteur est très important et doit répondre à un certain nombre de critères.

Tout d'abord, ce polymère doit pouvoir être déposé par les techniques de microdéposition citées précédemment, par l'intermédiaire d'une solution du polymère dans un solvant, de manière à former des gouttelettes calibrées et ajustées sur les microcuvettes. Un recuit à une température de 50 à 80°C pour éliminer le solvant et améliorer l'adhérence sur le substrat est généralement nécessaire.

Dans le cas idéal où le polymère est protecteur pendant les quatre étapes du cycle de synthèse, celui-ci doit être inerte et de préférence exempt de fonctions réactives capables de se coupler aux molécules et réactifs de synthèse. Il ne doit pas être soluble dans les solvants utilisés dans les étapes du cycle et présenter une imperméabilité vis-à-vis de ces solvants.

En réalité, il suffit que le polymère joue son rôle de couvercle protecteur essentiellement dans l'étape de détritylation ou dans l'étape de couplage. Il est possible alors d'adapter et d'optimiser le couple polymère-solvants pour l'étape retenue.

Il est préférable que le polymère de protection ne comporte pas de fonctions avec des hydrogènes libres du type OH, NH₂ ou COOH qui seraient capables de se coupler avec les molécules M1, M2, ... Mn, par exemple les nucléotides, au cours de l'étape de couplage, pour éviter de consommer inutilement des réactifs qui seront éliminés ensuite avec le polymère de protection.

Le polymère doit pouvoir être éliminé par un solvant inerte vis-à-vis des nucléotides et il doit de plus présenter des propriétés physico-chimiques en solution telles qu'une viscosité et une tension superficielle, permettant son dépôt sur les microcuvettes par la technique de microdéposition choisie.

Dans le procédé de l'invention, le choix du polymère de protection est effectué en fonction de la (des) étape(s) pendant lesquelles il doit protéger les microcuvettes, en tenant compte de sa solubilité dans les solvants susceptibles d'être utilisés pour la synthèse des oligonucléotides.

Si l'on veut assurer la protection par le polymère uniquement pendant l'étape d'élimination des groupes protecteurs des groupes fonctionnels, soit pendant l'étape de détritylation, il suffit de choisir un polymère insoluble dans les réactifs de détritylation qui sont généralement constitués par de l'acide dichloroacétique (DCA) ou trichloroacétique (TCA) ou un acide de Lewis du type ZnBr₂ , à environ 2-5 % dans un solvant tel que le dichlorométhane, l'acétonitrile ou le toluène.

A titre d'exemples de tels polymères, on peut citer les alcools polyvinyliques insolubles dans le dichlorométhane, l'acétonitrile et le toluène ; les polyhydroxystyrènes insolubles dans le dichlorométhane ; les polystyrènes, les polyvinylcarbazoles et les polyimides insolubles dans l'acétonitrile ; et les poly(oxyde d'éthylène) insolubles dans le toluène.

Si l'on veut assurer une protection des microcuvettes par le polymère seulement pendant l'étape de couplage, il convient de choisir un polymère insoluble dans le solvant utilisé pour le couplage, généralement l'acétonitrile.

Dans ce but, on peut utiliser un polymère choisi dans le groupe constitué de polymères et de leurs dérivés du type des alcools polyvinyliques, des polystyrènes, des polyvinylcarbazoles et des polyimides qui sont insolubles dans ce solvant.

Le polymère de protection est choisi également en fonction des solvants susceptibles d'assurer son élimination par dissolution. De préférence, on choisit des polymères solubles dans des solvants compatibles avec ceux utilisés habituellement dans les synthétiseurs automatiques d'oligonucléotides, qui sont généralement le dichlorométhane (pour la détritylation), l'acétonitrile (pour le couplage proprement dit) et le tétrahydrofuranne (pour le blocage et l'oxydation). Toutefois, d'autres solvants tels que l'acétonitrile et le toluène, peuvent être utilisés à la place du dichlorométhane pour la détritylation, sans perte de rendement.

Dans le tableau 1 qui suit, on a donné les polymères susceptibles d'être utilisés, leurs propriétés de solubilité dans divers solvants, leur utilisation possible dans une ou plusieurs étapes du procédé de l'invention et leur mode d'élimination.

**Tableau 1**

| **POLYMERE** | **SOLUBILITE** **(solvants)** | **INSOLUBILITE** **(solvants)** | **ETAPES POSSIBLES POUR PROTECTION** | **ELIMINATION** |
|---|---|---|---|---|
| **PVA** Polyvinylalcool | Eau, DMSO | CH₃CN ; THF ; CH₂Cl₂, Toluène | Détritylation et couplage dans CH₃CN, CH₂Cl₂, ou toluène Blocage et oxydation dans THF | Elimination dans l'eau ou DMSO |
| Polystyrène (PS) | Toluène Acétone CH₂Cl₂ | CH₃CN | - détritylation dans CH₃CN - couplage dans CH₃CN | Elimination dans CH₂Cl₂ |
| Polyhydroxystyrène (PHS) ou résine Shipley XP 8843 | CH₃CN THF, Alcool, Acétone, PGMA | CH₂Cl₂ | Détritylation dans CH₂Cl₂ | Elimination dans CH₃CN |
| Polyéthylèneoxyde (PEO) | CH₃CN CH₂Cl₂ Eau | THF, toluène | Détritylation dans le Toluène | Elimination dans eau CH₃CN CH₂Cl₂ |
| Polyvinylcarbazole (PVK) | CH₂Cl₂ Chlorobenzène | CH₃CN | Détritylation et couplage dans CH₃CN | Elimination dans CH₂Cl₂ |
| Polyimide XU 5218 (Ciba Geigy) | Anisole et CH₂Cl₂ | CH₃CN | Détritylation dans CH₃CN et couplage dans CH₃CN | Elimination dans CH₂Cl₂ |

Parmi les polymères du tableau 1, l'alcool polyvinylique est intéressant car il peut assurer une protection des microcuvettes pendant toutes les étapes de la synthèse d'oligonucléotides. De plus, il est facilement utilisable dans les robots de microdéposition. En revanche, son élimination est moins aisée car l'eau et le DMSO ne font pas partie des solvants de synthèse et nécessiteront de ce fait une étape complémentaire ainsi qu'un séchage.

Ainsi, dans le cas de la réalisation des sondes par utilisation d'un synthétiseur automate (support d'échantillon adapté) couplé à un robot microdispenseur (par capillarité, piézo-électrique ou du type «pin and ring»), il est préférable de ne pas utiliser un polymère protecteur soluble dans l'eau, qui nécessiterait à la fois un dépôt et une étape d'élimination en milieux aqueux ; ceci peut être gênant pour la poursuite de la synthèse qui est réalisée en milieu anhydre.

Pour éviter tout traitement de séchage supplémentaire, il est plus judicieux de choisir pour la protection sélective des microcuvettes un polymère qui sera éliminé dans un solvant anhydre et compatible avec le cycle des solvants utilisés dans le synthétiseur automate.

Par exemple, le polystyrène peut servir de polymère de protection sélective dans l'étape de détritylation réalisée dans i'acétonitrile (TCA 2%) dans lequel il est insoluble ; l'étape suivante de couplage se fait dans l'acétonitrile (tétrazole) ; le polymère peut être alors éliminé par un cycle de rinçage dans le dichlorométhane dans lequel il est très soluble.De plus, le polystyrène est inerte et ne présente pas des fonctions OH capables de fixer des nucléotides actifs et il peut jouer également son rôle protecteur dans l'étape de couplage (dans l'acétonitrile).

Le polyhydroxystyrène est lui aussi intéressant car il est plus facile à déposer que le polystyrène et facilement éliminé dans un solvant classique du synthétiseur. En revanche, il ne peut assurer la protection que pendant l'étape de détritylation dans le dichlorométhane mais peut être éliminé par un cycle de rinçage dans l'acétonitrile avant l'étape de couplage dans ce solvant.

Le poly(oxyde d'éthylène) ne convient lui aussi que pour la détritylation à condition d'effectuer celle-ci dans le toluène, mais il est facilement éliminé dans CH₃CN ou CH₂Cl₂.

Le polyvinylcarbazole et le polyimide peuvent assurer une protection pendant les étapes de détrilytation et de couplage dans CH₃CN, puis être éliminés au moyen de CH₂Cl₂ qui fait partie des solvants utilisés dans les synthétiseurs. Cependant, le dépôt de ces polymères est plus difficile.

L'élimination du polymère de protection peut se faire directement dans le synthétiseur, dans une étape de rinçage, introduite dans le protocole du cycle du synthétiseur automatique.

Par exemple le solvant classique utilisé dans l'étape de détritylation, le dichlorométhane peut être remplacé par des solvants comme l'acétonitrile , le toluène, le xylène, ou encore un solvant aromatique halogéné, sans perte d'efficacité de la réaction de détritylation par le réactif acide utilisé habituellement (TCA ou DCA).

Le procédé de l'invention présente de nombreux avantages par rapport aux procédés de l'art antérieur.

En effet, par rapport au procédé de la référence [2] qui utilise un adressage photochimique pour la synthèse in situ ou celui de la référence [4] qui utilise un procédé lithographique, le procédé de l'invention évite la nécessité de réaliser des masques, c'est-à-dire des opérations longues et coûteuses, car il s'agit de réaliser un nombre de masques égal au nombre de molécules M1, M2, ... Mn qui constituent les séquences. De plus, dans le cas de l'adressage photochimique, la déprotection n'est pas totale et plus la séquence est longue, plus la probabilité d'obtenir la séquence voulue est faible. De ce fait, on constitue sur le substrat des séquences redondantes, ce qui n'est pas nécessaire dans l'invention où la déprotection est totale.

Par rapport au procédé de la référence [1], le procédé de l'invention est plus facile à mettre en oeuvre car les réactions de couplage peuvent être effectuées avec un excès de réactifs alors que, selon la référence [1], les réactions de couplages sont effectuées avec quelques nl de réactifs directement sur le site à modifier dans des conditions particulières, soit en atmosphère d'azote ou d'argon.

Par rapport à la référence [3], le procédé de l'invention est plus facile à mettre en oeuvre car les microcuvettes, ou sites de synthèse, sont définis préalablement, la protection est plus étanche par mise en place de couvercles de polymère solide et les réactions de déprotection peuvent être effectuées en phase liquide directement dans un synthétiseur automatique, et non pas en phase vapeur.

Dans le procédé de l'invention, le dépôt du polymère de protection sur les microcuvettes sélectionnées par microdéposition ne nécessite pas de précautions particulières, la seule condition étant que la gouttelette de polymère recouvre la microcuvette à protéger.

D'autres caractéristiques et avantages de l'invention apparaîtront mieux à la lecture de la description qui suit, donnée bien entendu à titre illustratif et non limitatif en référence aux dessins annexés.

### Brève description des dessins

Les figures 1A à 1G illustrent les différentes étapes du procédé de l'invention pour la réalisation d'une matrice de sondes d'oligonucléotides.
La figure 2 illustre le profil d'une microcuvette de (650 x 650 x 24 µm³) remplie de polymère.
Les figures 3 et 4 sont des vues de dessus de microcuvettes remplies ou non de polymère.

### Exposé détaillé des modes de réalisation

Pour mettre en oeuvre le procédé de l'invention, on part d'un substrat en verre ou de préférence en silicium, structuré avec des microcuvettes au pas de 150 µm à quelques mm, mais de préférence quelques centaines de µm et de profondeur de l'ordre de 10 µm. Ces microcuvettes calibrées sont réalisées par des méthodes classiques de photolithographie et de gravure.

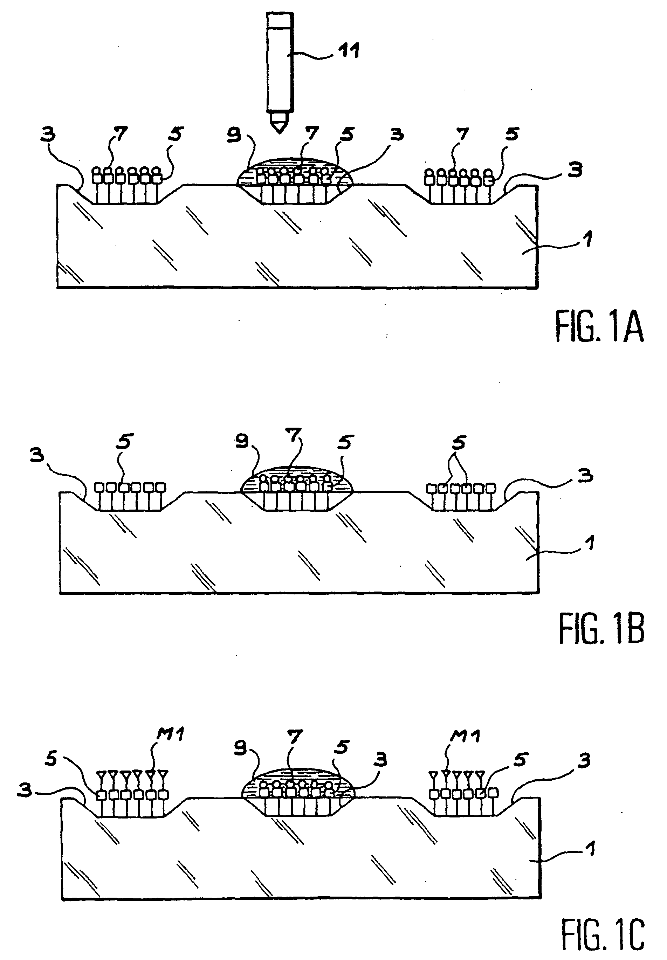

Sur la figure 1A, on voit le substrat 1 muni des microcuvettes 3. Les microcuvettes 3 sont fonctionnalisées par des groupes fonctionnels hydrophiles 5 tels que des groupes hydroxyles, qui sont protégés par des groupes protecteurs 7. Ces groupes protecteurs peuvent être des groupes diméthoxytrityle, habituellement utilisés en synthèse oligonucléotidique.

Lorsque le substrat est une plaquette en silicium; la fonctionnalisation des sites peut être effectuée de la façon suivante.

La surface de la plaquette en silicium est tout d'abord oxydée en SiO₂ par voie thermique, par exemple sur une épaisseur de 5000 Å, puis activée par nettoyage oxydant en milieu alcalin ou acide en Si-OH. Ensuite, on procède à une fonctionnalisation de la surface totale du substrat par traitement avec un agent de silanisation fonctionnalisé du type aminosilane ou époxysilane avec une molécule de liaison ou bras espaceur du type glycol, par exemple un silane comportant un groupement N(bis-hydroxyméthyle) et un bras espaceur par exemple du type polyéthylène glycol. L'introduction d'un tel espaceur entre le silane du substrat et l'oligonucléotide qui sera synthétisé par la suite est intéressante car elle permet d'augmenter, lors de l'utilisation, le rendement de l'hybridation. On protége les fonctions réactives par. un groupe tel que le groupe diméthoxytrityle (DMT).

On réalise alors une protection de l'ensemble des microcuvettes par des couvercles de polymère déposé sous forme de gouttelettes calibrées par un robot de microdéposition par jets.

Après séchage des gouttelettes, on déprotège par détritylation en milieu acide (TCA) les fonctions ODMT de la surface du substrat non protégé par le polymère. On bloque (capping) ces fonctions réactives de la surface extérieure aux microcuvettes afin de limiter la synthèse in situ des oligonucléotides uniquement dans les microcuvettes, puis on élimine le polymère sur l'ensemble du substrat par rinçage dans un solvant.

On réalise ensuite l'étape 2) de dépôt d'un polymère de protection 9 sur au moins l'une des microcuvettes fonctionnalisées de la plaquette, à l'aide d'une micropipette 11 telle qu'un robot dispenseur ou par impression par jets, comme représenté sur la figure 1A.

Sur la figure 1B, on a représenté l'étape suivante qui consiste à éliminer les groupes protecteurs 7 des groupes fonctionnels 5 sur les microcuvettes 3 de la plaquette non recouvertes du polymère de protection 9. On obtient ainsi une plaquette dont les microcuvettes 3 non protégées par le polymère de protection 9, comportent des groupes fonctionnels hydroxyle 5 déprotégés. Cette étape peut être réalisée en trempant la plaquette dans une solution acide, par exemple une solution d'acide trichloroacétique dans du dichlorométhane CH₂Cl₂, pour éliminer les groupes trityle.

Sur la figure 1C, on a représenté l'étape suivante 3) de couplage d'une molécule M1 par réaction avec les groupes fonctionnels hydroxyle 5 sur les microcuvettes 3 non protégées par le polymère de protection 9. Dans le cas où l'on veut réaliser une matrice de sondes oligonucléotidiques, les molécules M1 sont constituées par des nucléotides tels que des phosphoramidites, des phosphonates ou des phosphites selon le mode de synthèse utilisé pour la fabrication de la sonde. Dans cette étape, on réalise le couplage du premier nucléotide M1 (phosphoramidite) avec les groupes hydroxyle 5 en immergeant la plaquette dans une solution du nucléotide M1 activé dans l'acétonitrile en présence de tétrazole. On obtient ainsi la structure représentée sur la figure 1C.

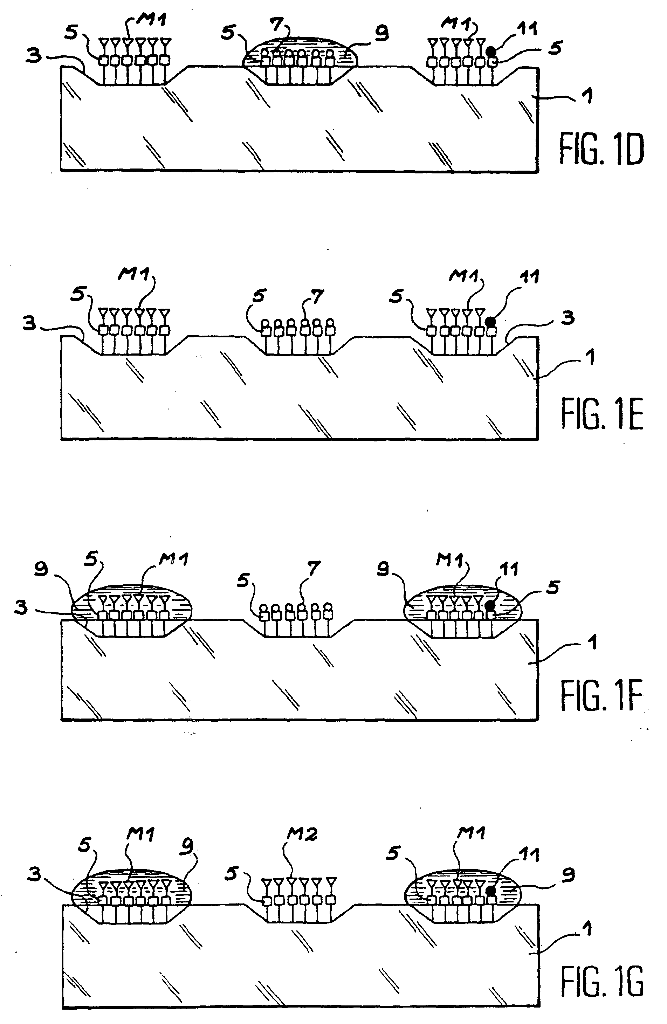

Sur la figure 1D, on a représenté l'étape suivante, selon laquelle on bloque les groupes hydroxyle libres restants qui n'auraient pas réagi avec la molécule M1, afin qu'ils ne puissent réagir ensuite avec les autres nucléotides utilisés pour les différents couplages successifs. Cette réaction peut être effectuée par mise en place d'un groupe bloquant 11, par exemple par trempage de la plaquette dans une solution de diméthylaminopyridine DMAP dans du tétrahydrofuranne THF.

Dans le cas de la fabrication d'une séquence oligonucléotidique par la méthode utilisant des phosphoramidites, on procède ensuite à une oxydation du phosphore trivalent introduit lors du couplage de l'étape précédente (figure 1C), en phosphore pentavalent plus stable. Ceci peut être réalisé par trempage de la plaquette dans une solution oxydante d'iode dans un mélange d'eau, de pyridine et de tétrahydrofuranne THF.

Après cette étape d'oxydation, on réalise l'étape suivante 4) d'élimination du polymère de protection 9 sur les microcuvettes non modifiées. On obtient ainsi la structure représentée sur la figure 1E. Cette élimination du polymère protecteur 9 peut être réalisée par dissolution dans un solvant approprié en trempant la plaquette dans ce solvant.

Ainsi, mis à part l'étape de dépôt du polymère de protection, les étapes du procédé de l'invention sont réalisées par trempage de la plaquette dans les réactifs et solvants appropriés, suivi de rinçages dans différents solvants, si nécessaire. Ces opérations peuvent se faire dans des récipients avec atmosphère contrôlée. Ces différentes étapes par trempage dans les bains des réactifs appropriés peuvent être avantageusement réalisées dans une cellule réactionnelle, connectée à un synthétiseur automatique d'oligonucléotides.

De plus, les réactifs utilisés sont en grand excès par rapport aux molécules sur le substrat solide, ce qui présente l'avantage d'obtenir des réactions avec un taux de conversion pratiquement de 100 %, ce qui n'était pas le cas avec le procédé de la référence [1] où les réactions sont effectuées par micropipettage des réactifs sur les sites choisis.

Après cette première étape de modification des microcuvettes choisies par le nucléotide M1, on peut modifier d'autres microcuvettes avec une molécule M2, ... ou Mn. Dans ce cas, on procède tout d'abord à une protection des microcuvettes déjà modifiées par le polymère de protection 9. On obtient ainsi la structure représentée sur la figure 1F. Bien évidemment, d'autres microcuvettes du substrat peuvent être également recouvertes du polymère de protection 9 même si elles n'ont pas été modifiées dans les étapes précédentes par la molécule M1. Après cette opération, on élimine alors les groupes protecteurs 7 des groupes fonctionnels hydrophiles 5 des microcuvettes non recouvertes du polymère de protection 9, puis on couple sur ces groupes fonctionnels 5 un autre nucléotide M2 en opérant de la même façon que précédemment (figures 1B et 1C). La figure 1G illustre la structure obtenue. Après cette opération, on peut procéder au blocage des groupes hydroxyle n'ayant pas réagi, puis à une oxydation du phosphore trivalent en phosphore pentavalent, et à l'élimination du polymère protecteur 9 sur les microcuvettes non modifiées par la molécules M2.

On réalise ensuite de nouveau ces différentes opérations sur les microcuvettes choisies pour obtenir l'enchaînement voulu de molécule M1, M2, ... Mn, soit des oligonucléotides d'enchaînements différents sur des microcuvettes différentes du substrat 1.

A titre d'exemple, on a réalisé une matrice de sondes oligonucloéotidiques sur un substrat en silicium comportant des microcuvettes de 100 x 100 x 30 µm gravées dans le silicium. La fonctionnalisation des microcuvettes a été effectuée avec un agent de silanisation constitué par du N,N-(bis-hydroxyéthyl)aminopropyl triméthoxysilane dont les groupes OH sont protégés par un groupe diméthoxy ou monométhoxy trityle.

On décrit ci-après un exemple de réalisation d'une matrice de deux séquences d'oligonucléotides.

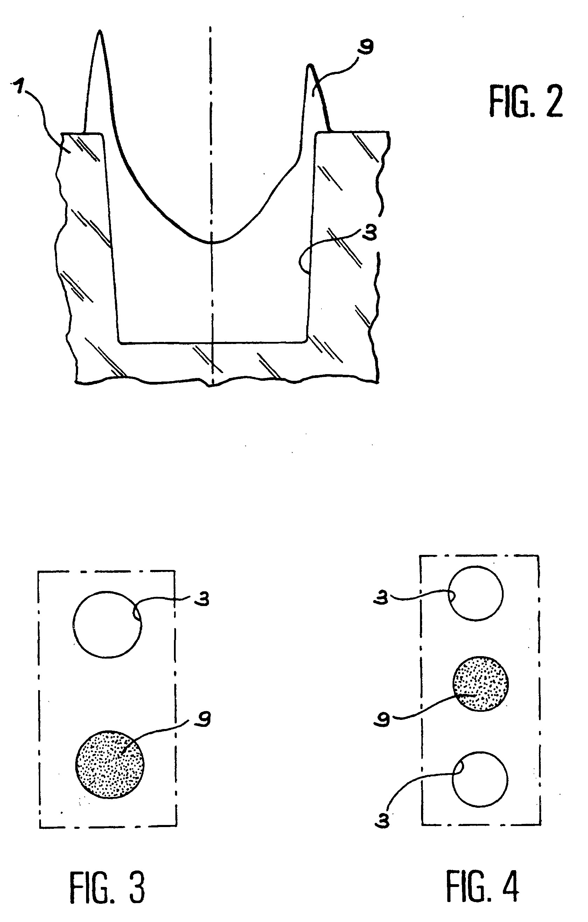

On part d'un substrat structuré avec des microcuvettes par utilisation des méthodes classiques de microélectronique à savoir, dépôt, photolithographie et gravure. Un profil de microcuvette 3 est montré sur la figure 2.

Après découpe d'un substrat de silicium en puce de 2 x 2 cm², les protocoles de nettoyage (1), silanisation (2) et branchement d'un bras hexaéthylène glycol (3) sont effectués de la manière suivante :
- 1) Le substrat est incubé pendant 2 heures à température ambiante dans une solution de NaOH (lg NaOH, 3 ml d'eau désionisée DI, 4 ml EtOH 95%), puis rincé à l'eau DI et séché avec une soufflette
- 2) Le substrat est incubé une nuit à 80°C dans une solution comportant 1 ml de 3-glycidoxypropyltriméthoxysilane, 3,5 ml de toluène et 0,3 ml de triéthylamine. Il est rincé à l'acétone, séché à la soufflette, puis mis 3 heures à 110°C.
- 3) Le substrat est incubé une nuit à 80°C dans une solution contenant 15 ml d'hexaéthylène glycol et 9 µl d'acide sulfurique, puis il est rincé dans l'acétone et séché à la soufflette.

Le substrat est ensuite positionné dans une pièce spécifique reliée à un synthétiseur automatique d'oligonucléotides EXPEDITE 8909. Le protocole de synthèse utilise la chimie phosphoramidite et comporte quatre étapes : détritylation, couplage, capping, oxydation. Le protocole utilisé est à l'échelle de 1 µmole.

Les sept premiers mères sont effectués dans le synthétiseur automatique 3' ATC TCA C 5' Le substrat est sorti du synthétiseur et est amené sur un robot de dépôt de polymère (Cam/alot) ou le polymère est dispensé sur la moitié des cuvettes. Le profil du remplissage de polymère 9 dans la microcuvette 3 est représenté sur la figure 2. Une vue de dessus des microcuvettes 3 au microscope optique est montré sur la figure 3 pour des tailles de 300 µm au pas de 600 µm, et sur la figure 4 pour des tailles de 200 µm au pas de 400 µm. Certaines microcuvettes sont vides.

Le substrat est cuit à 85°C pendant 1 min 30's sur une platine chauffante. Le substrat est remis dans le synthétiseur pour effectuer le couplage d'une base C sur la moitié des cuvettes non recouvertes par le polymère. Le polymère est éliminé dans l'eau à 85°C. Un nouveau dépôt de polymère est effectué sur ces dernières cuvettes. Après cuisson, le substrat est positionné dans le synthétiseur pour effectuer le couplage d'une base T dans les autres cuvettes. Après ce couplage, le polymère est éliminé et le substrat est positionné dans le synthétiseur où l'ensemble des cuvettes reçoit les couplages suivants : C AAA TAG. A la fin de cette étape la moitié des microcuvettes contient la séquence NO 1 : -3' ATC TCA CTC AAA TAG 5'- et l'autre moitié la séquence NO 2 : -3' ATC TCA CCC AAA TAG 5'-.

Ces deux séquences se différencient seulement par une seule base. Le substrat est sorti du synthétiseur et il est incubé dans une solution de NH₄OH pendant 45 min à 60°C, puis il est rincé à l'eau DI et séché à la soufflette afin d'éliminer les groupements protecteurs des bases nucléotidiques.

L'hybridation est effectuée avec la cible complémentaire de la sonde n°2 : -3'CA TAG AGT GGG TTT ATC CA 5' comportant un groupement biotine en 5'. Le substrat est mis dans une solution contenant 690 µl de tampon d'hybridation H-7140 de Sigma et 10 µl de la cible à 1 OD (quantité d'oligonucléotides qui, lorsqu'ils sont dissous dans 1 ml d'eau, résulte en une absorbance de 1 mesurée à 260 nm dans une cuve de trajet optique égal à 1 cm.) et il est incubé à 40°C pendant 1 heure avec une légère agitation, puis on effectue 2 rinçages dans un bain de tampon SCC 2X pendant 1 minute, et dans un bain de tampon SCC 0,2X pendant 1 minute afin d'éliminer les cibles non hybridées. Le substrat est alors séché à la soufflette. Le couplage de la biotine avec le groupement streptavidine comportant le groupement fluorophore Cy3 se fait dans une solution contenant 5 µl de tampon de Streptavidine Cy3 et 700 µl de tampon PBS, TW, NaCl 0,5 M à température ambiante dans l'obscurité pendant 10 à 15 minutes. Le substrat est ensuite rincé dans un bain de tampon PBS, TW, NaCl 0,5M pendant 1 minute, puis dans un bain de tampon PBS et séché à la soufflette.

L'image de fluorescence obtenue après hybridation sur le système microscope confocal à fluorescence GS 3000 indique que seule l'hybridation a lieu avec la sonde complémentaire.

### Références citées

[1] : US-A-5 474 796
[2] : WO-A-97/39 151
[3] : EP-A-0 728 520
[4] : US-A-5 658 734

## Revendications

1. Procédé de réalisation d'une matrice de séquences de molécules chimiques ou biologiques formées d'enchaînements différents de molécules M1, M2, ... Mn, par synthèse in situ sur un substrat structuré avec des microcuvettes, comprenant les étapes suivantes :
1) fonctionnalisation des microcuvettes du substrat par des groupes fonctionnels capables de former une liaison covalente avec les molécules M1, M2, ... Mn ;
2) dépôt d'un polymère de protection dans au moins l'une des microcuvettes par microdéposition de gouttes dudit polymère pour constituer dans la(les) microcuvette(s) sélectionnée(s) des couvercles de polymère solide ;
3) réalisation d'une ou plusieurs réactions chimiques en phase liquide pour assurer le couplage d'une première molécule M1 sur les groupes fonctionnels des microcuvettes non recouvertes de polymère de protection ;
4) élimination du polymère de protection sur les microcuvettes recouvertes de ce polymère, après la première réaction du l'une des réactions suivantes effectuées dans l'étape 3) ; et
5) réalisation à nouveau des étapes 2), 3) et 4) pour obtenir les séquences d'enchaînement voulu sur chacune des microcuvettes fonctionnalisées ;
dans lequel l'étape 1) consiste :
a) à fonctionnaliser la surface totale du substrat et des microcuvettes par les groupes fonctionnels, puis à protéger ces groupes fonctionnels par un groupe ,protecteur labile ;
b) à déposer un polymère de protection sur toutes les microcuvettes par microdéposition de gouttes dudit polymère pour constituer des couvercles de polymère solide sur toutes les microcuvettes ;
c) à déprotéger les groupes fonctionnels présents sur le substrat autour des microcuvettes, puis à les faire réagir avec un groupe bloquant non labile dans les conditions utilisées pour les réactions chimiques de couplage et pour l'élimination du polymère de protection ; et
d) à éliminer le polymère de protection de toutes les microcuvettes ;
et dans lequel le polymère de protection étant déposé à partir d'une solution dans un solvant, on recuit le film de polymère formé par chauffage du substrat à une température de 50 à 100°C avant d'effectuer l'étape 3) ou l'étape c).

2. Procédé selon la revendication 1, dans lequel on protège les groupes fonctionnels des microcuvettes par un groupe protecteur labile, et dans lequel l'étape 3) comprend comme première réaction, une réaction de déprotection des groupes fonctionnels.

3. Procédé selon la revendication 1 ou 2, dans lequel le groupe protecteur est un groupe trityle ou dérivé de trityle.

4. Procédé selon l'une quelconque des revendications 1 à 3, dans lequel les séquences de molécules sont des oligonucléotides.

5. Procédé selon la revendication 3 ou 4, dans lequel l'étape 3) est un cycle de synthèse phosphoramidite d'oligonucléotides, qui comprend l'élimination des groupes trityle, le couplage avec un nucléotide, la réaction des groupes fonctionnels qui n'ont pas été couplés au nucléotide, avec un groupe bloquant, et l'oxydation en phosphate du groupement phosphoramidite du nucléotide.

6. Procédé selon la revendication 5, dans lequel on réalise l'étape 4) d'élimination du polymère de protection après couplage du nucléotide.

7. Procédé selon la revendication 6, dans lequel le polymère de protection est choisi dans le groupe constitué de polymères et de leurs dérivés du type des alcools polyvinyliques, des polystyrènes, des polyvinylcarbazoles et des polyimides.

8. Procédé selon la revendication 5, dans lequel on réalise l'étape 4) d'élimination du polymère de protection, après l'étape 3) d'élimination des groupes trityle.

9. Procédé selon la revendication 8, dans lequel le polymère de protection est un polyhydroxystyrène et la détritylation est effectuée dans du dichlorométhane.

10. Procédé selon la revendication 8, dans lequel le polymère de protection est un polystyrène ou un polyvinylcarbazole, et la détritylation est effectuée dans l'acétonitrile.

11. Procédé selon la revendication 8, dans lequel le polymère de protection est un poly(oxyde d'éthylène), et la détritylation est effectuée dans du toluène.

12. Procédé selon la revendication 8, dans lequel le polymère de protection est un alcool polyvinylique, et la détritylation est effectuée dans du dichlorométhane.

13. Procédé selon l'une quelconque des revendications 8 à 12, dans lequel l'étape 4) d'élimination du polymère de protection est effectuée par rinçage au moyen d'un solvant du polymère en ajoutant cette étape de rinçage dans le cycle d'un synthétiseur automatique d'oligonucléotides.

14. Procédé selon la revendication 1, dans lequel on dépose le polymère de protection au moyen d'un robot de microdéposition par la technique d'impression par jets d'encres ou de dispenseur de microgouttes calibrées.

## Patentansprüche

1. Verfahren zur Herstellung einer Matrix aus chemischen oder biologischen Molekülsequenzen, die durch Verkettung verschiedener Moleküle M1, M2, ... Mn gebildet worden sind, durch in situ-Synthese auf einem strukturierten Substrat mit Mikroküvetten, das die folgenden Stufen umfasst:
1) Funktionalisierung der Mikroküvetten des Substrats durch funktionelle Gruppen, die mit den Molekülen M1, M2, ... Mn eine kovalente Bindung bilden können;
2) Abscheidung eines Schutzpolymers in mindestens einer der Mikroküvetten durch Mikroabscheidung von Tropfen des Polymers zur Bildung von Abdeckungen (Überzügen) aus dem festen Polymer in der (den) ausgewählten Mikroküvette(n);
3) Durchführung einer oder mehrerer chemischer Reaktionen in flüssiger Phase, um die Kupplung eines ersten Moleküls M1 an die funktionellen Gruppen der Mikroküvetten, die nicht von dem Schutzpolymer bedeckt sind, zu gewährleisten;
4) Eliminierung des Schutzpolymers auf den Mikroküvetten, die von diesem Polymer bedeckt sind, nach der ersten Reaktion oder einer der nachfolgenden Reaktionen, die in der Stufe (3) durchgeführt werden; und
5) Wiederholung der Stufen (2), (3) und (4) zur Erzielung der gewünschten Verkettungssquenzen an jeder der funktionalisierten Mikroküvetten, wobei in dem Verfahren die Stufe (1) darin besteht, dass man:
a) die gesamte Oberfläche des Substrats und der Mikroküvetten mit funktionellen Gruppen funktionalisiert, dann diese funktionellen Gruppen durch eine labile Schutzgruppe schützt;
b) ein Schutzpolymer auf allen Mikroküvetten durch Mikroabscheidung von Tropfen des Polymers abscheidet zur Bildung von Überzügen aus dem festen Polymer auf allen Mikroküvetten;
c) die auf dem Substrat um die Mikroküvetten herum vorhandenen funktionellen Gruppen von der Schutzgruppe befreit, dann mit einer nichtlabilen blockierenden Gruppe reagieren lässt unter den Bedingungen, wie sie für die chemischen Reaktionen zur Kupplung und zur Eliminierung des Schutzpolymers angewendet worden sind, und
d) das Schutz-Polymer aus allen Mikroküvetten eliminiert; und
bei dem Schutz-Polymer, das ausgehend von einer Lösung in einem Lösungsmittel abgeschieden worden ist, den gebildeten Polymer-Film tempert durch Erwärmen des Substrats auf eine Temperatur von 50 bis 100 °C, bevor die Stufe (3) oder die Stufe (c) durchgeführt wird.

2. Verfahren nach Anspruch 1, bei dem man die funktionellen Gruppen der Mikroküvetten durch eine labile Schutzgruppe schützt und bei dem die Stufe (3) als erste Reaktion eine Reaktion zur Entfernung der funktionellen Schutzgruppen umfasst.

3. Verfahren nach Anspruch 1 oder 2, bei dem die Schutzgruppe eine Tritylgruppe oder ein Tritylderivat ist.

4. Verfahren nach einem der Ansprüche 1 bis 3, bei dem die Molekülsequenzen Oligonucleotide sind.

5. Verfahren nach Anspruch 3 oder 4, bei dem die Stufe (3) ein Phosphoramidit-Zyklus zur Synthese von Oligonucleotiden ist, der die Eliminierung der Tritylgruppen, das Kuppeln mit einem Nucleotid, die Reaktion der funktionellen Gruppen, die nicht an das Nucleotid gekoppelt worden sind, mit einer blockierenden Gruppe und die Oxidation der Phosphoramidit-Gruppe des Nucleotids zu einem Phosphat umfasst.

6. Verfahren nach Anspruch 5, bei dem man die Stufe (4) zur Eliminierung des Schutz-Polymers nach dem Kuppeln des Nucleotids durchführt.

7. Verfahren nach Anspruch 6, bei dem man das Schutz-Polymer auswählt aus der Gruppe, die besteht aus Polymeren und ihren Derivaten vom Polyvinylalkohol-Typ, Polystyrolen, Polyvinylcarbazolen und Polyimiden.

8. Verfahren nach Anspruch 5, bei dem man die Stufe (4) zur Eliminierung des Schutz-Polymers nach der Stufe (3) zur Eliminierung der Tritylgruppen durchführt.

9. Verfahren nach Anspruch 8, bei dem das Schutz-Polymer ein Polyhydroxystyrol ist und die Detritylierung in Dichlormethan durchgeführt wird.

10. Verfahren nach Anspruch 8, bei dem das Schutz-Polymer ein Polystyrol oder ein Polyvinylcarbazol ist und die Detritylierung in Acetonitril durchgeführt wird.

11. Verfahren nach Anspruch 8, bei dem das Schutz-Polymer ein Poly(oxyethylen) ist und die Detritylierung in Toluol durchgeführt wird.

12. Verfahren nach Anspruch 8, bei dem das Schutz-Polymer ein Polyvinylalkohol ist und die Detritylierung in Dichlormethan durchgeführt wird.

13. Verfahren nach einem der Ansprüche 8 bis 12, bei dem die Stufe (4) zur Eliminierung des Schutz-Polymers durch Spülen mit einem Lösungsmittel für das Polymer durchgeführt wird, indem man diese Spülstufe in den Zyklus eines automatischen Oligonucleotid-Synthetisators einfügt.

14. Verfahren nach Anspruch 1, bei dem man das Schutz-Polymer unter Verwendung eines Mikroabscheidungs-Roboters durchführt durch Tintenstrahldrucken oder Ausgabe von geeichten Mikrotropfen.

## Claims

1. Method for producing a template of sequences of chemical or biological molecules formed from different chains of molecules, M1, M2, ..., Mn, by in situ synthesis on a structured substrate with microcuvettes, comprising the following steps:
1) functionalization of the microcuvettes of the substrate by functional groups capable of forming a covalent bond with the molecules M1, M2, ..., Mn;
2) deposition of a protection polymer on at least one of the microcuvettes by microdeposition of drops of said polymer to form caps of solid polymer on the selected microcuvette(s);
3) carrying out one or more chemical reactions to provide coupling of a first molecule M1 on the functional groups of the microcuvettes not covered with protection polymer;
4) removal of the protection polymer on the microcuvettes covered with this polymer, after the first reaction or one of the following reactions carried out in step 3); and
5) again carrying out steps 2), 3) and 4) to obtain the desired chaining sequences on each of the functionalized microcuvettes;
wherein step 1) comprises:
a) functionalizing the total surface of the substrate and the microcuvettes by functional groups, then protecting said functional groups by a labile protection group;
b) depositing a protection polymer on all the microcuvettes by the microdeposition of drops of said polymer in order to form solid polymer caps on all the microcuvettes;
c) deprotecting the functional groups present on the substrate around the microcuvettes, then reacting them with a non-labile blocking group under conditions used for the chemical coupling reactions and for the elimination of the protection polymer; and
d) eliminating the protection polymer from all the microcuvettes;
and wherein the protection polymer is deposited from a solution in a solvent, the polymer film formed being annealed by heating the substrate to a temperature of 50 to 10°C prior to carrying out step 3) or step c).

2. Method according to claim 1, wherein functional groups of the microcuvettes are protected by a labile protective group and wherein step 3) comprises as the first reaction a deprotection reaction of the functional groups.

3. Method according to claim 1 or 2, wherein the protective group is a trityl or trityl-derived group.

4. Method according to any of claims 1 to 3, wherein the sequences of molecules are oligonucleotides.

5. Method according to claims 3 and 4, wherein step 3) is a phosphoramidite synthesis cycle of oligonucleotides which consists of removing the trityl groups, coupling with the nucleotide, reacting the functional groups, which have not been coupled with the nucleotide, with a blocking group and oxidizing the phosphoramidite group of the nucleotide into a phosphate.

6. Method according to claim 5, wherein step 4) is carried out with removal of the protection polymer after coupling of the nucleotide.

7. Method according to claim 6, wherein the protection polymer is selected from the group formed by polymers and their derivatives of polyvinyl alcohols, polystyrenes, polyvinyl carbazoles and polyimides.

8. Method according to claim 5, wherein step 4) is carried out for removing the protection polymer, after step 3) for removing the trityl groups.

9. Method according to claim 8, wherein the protection polymer is polyhydroxystyrene and detritylation is carried out in dichloromethane.

10. Method according to claim 8, wherein the protection polymer is polystyrene or polyvinyl carbazole, and detritylation is carried out in acetonitrile.

11. Method according to claim 8, wherein the protection polymer is polyethylene oxide and detritylation is carried out in toluene.

12. Method according to claim 8, wherein the protection polymer is polyvinyl alcohol and detritylation is carried out in dichloromethane.

13. Method according to any one of claims 8 to 12, wherein step 4) for removal of the protection polymer is carried out by rinsing by means of a solvent of the polymer by adding this rinsing step in the cycle of an automatic synthesizer of oligonucleotides.

14. Method according to claim 1, wherein the protection polymer is deposited by means of a microdeposition robot by the ink jet printing technique or dispenser of calibrated microdrops.
